# EUROPEAN PATENT SPECIFICATION

(11) **EP 3 717 075 B1**
(45) Date of publication and mention of the grant of the patent: **30.10.2024**
(21) Application number: 18826826.2
(22) Date of filing: 14.11.2018
(51) Int. Cl.: A61Q 17/04, A61K 8/81, A61K 8/92, A61K 8/03, A61K 8/04

(54) **A BI-PHASE SUN CARE COMPOSITION**
ZWEIPHASIGE SONNENSCHUTZMITTEL
COMPOSITION DE SOIN SOLAIRE BIPHASE

(30) Priority: 28.11.2017 JP 2017227809
(43) Date of publication of application: 07.10.2020
(73) Proprietor: L'OREAL, 75008 Paris (FR); Koike, Toru, Kanagawa 213-0012 (JP); Takahashi, Nozomi, Takatsu-ku Kawasaki-shi Kanagawa 213-0012 (JP)
(72) Inventor: KOIKE, Toru, Kawasaki-shi Kanagawa 213-0012 (JP); TAKAHASHI, Nozomi, Kawasaki-shi Kanagawa 213-0012 (JP)
(74) Representative: Casalonga
(86) International application number: PCT/JP2018/042913
(87) International publication number: WO 2019/107230

(56) References cited:
- EP-A2- 2 011 481
- WO-A1-03/070201
- WO-A1-2010/118415
- DE-C1- 4 426 952
- US-A1- 2004 005 279
- US-A1- 2007 009 453
- DATABASE GNPD [online] MINTEL; 25 September 2012 (2012-09-25), ANONYMOUS: "Water Sport Instant Dry Mist SPF 30", XP055559045, retrieved from https://www.gnpd.com/sinatra/recordpage/1875822/ Database accession no. 1875822
- DATABASE GNPD [online] MINTEL; 5 June 2017 (2017-06-05), ANONYMOUS: "Perfection Two-Phase Serum SPF15", XP055559036, retrieved from https://www.gnpd.com/sinatra/recordpage/4864163/ Database accession no. 4864163
- DATABASE GNPD [online] MINTEL; 1 September 2017 (2017-09-01), ANONYMOUS: "Body Guard SPF 30", XP055559048, retrieved from https://www.gnpd.com/sinatra/recordpage/5072799/ Database accession no. 5072799
- DATABASE GNPD [online] MINTEL; 9 June 2011 (2011-06-09), ANONYMOUS: "Anti-Jellyfish Sunscreen SPF 50+", XP055559041, retrieved from https://www.gnpd.com/sinatra/recordpage/1558554/ Database accession no. 1558554
- DATABASE GNPD [online] MINTEL; 22 August 2017 (2017-08-22), ANONYMOUS: "Sun Milk Spray SPF 30", XP055559053, retrieved from https://www.gnpd.com/sinatra/recordpage/5041261/ Database accession no. 5041261
- DATABASE GNPD [online] MINTEL; 27 July 2017 (2017-07-27), ANONYMOUS: "Transparent Sunscreen Spray SPF 20", XP055559050, retrieved from https://www.gnpd.com/sinatra/recordpage/4986553/ Database accession no. 4986553
- DATABASE GNPD [online] MINTEL; 26 September 2017 (2017-09-26), ANONYMOUS: "Trigger-Happy Spray SPF 50+", XP055559049, retrieved from https://www.gnpd.com/sinatra/recordpage/5129827/ Database accession no. 5129827
- DATABASE GNPD [online] MINTEL; 5 June 2017 (2017-06-05), ANONYMOUS: "Perfection Two-Phase Serum SPF15", XP055559036, retrieved from www.gnpd.com Database accession no. 4864163
- DATABASE GNPD [online] MINTEL; 9 June 2011 (2011-06-09), ANONYMOUS: "Anti-Jellyfish Sunscreen SPF 50+", XP055559041, retrieved from www.gnpd.com Database accession no. 1558554
- DATABASE GNPD [online] MINTEL; 25 September 2012 (2012-09-25), ANONYMOUS: "Water Sport Instant Dry Mist SPF 30", XP055559045, retrieved from www.gnpd.com Database accession no. 1875822
- DATABASE GNPD [online] MINTEL; 1 September 2017 (2017-09-01), ANONYMOUS: "Body Guard SPF 30", XP055559048, retrieved from www.gnpd.com Database accession no. 5072799
- DATABASE GNPD [online] MINTEL; 26 September 2017 (2017-09-26), ANONYMOUS: "Trigger-Happy Spray SPF 50+", XP055559049, retrieved from www.gnpd.com Database accession no. 5129827
- DATABASE GNPD [online] MINTEL; 27 July 2017 (2017-07-27), ANONYMOUS: "Transparent Sunscreen Spray SPF 20", XP055559050, retrieved from www.gnpd.com Database accession no. 4986553
- DATABASE GNPD [online] MINTEL; 22 August 2017 (2017-08-22), ANONYMOUS: "Sun Milk Spray SPF 30", XP055559053, retrieved from www.gnpd.com Database accession no. 5041261

## Description

### TECHNICAL FIELD

The present invention relates to a sun care composition, in particular a sun care cosmetic composition in a bi-phase form, for a keratinous substance such as skin.

### BACKGROUND ART

For sun care products, good UV protecting effects and good usability are among the key functions. In addition, in order to satisfy consumer's preference, it is preferable that sun care products show a clear appearance.

Bi-phase compositions comprising an aqueous phase and an oily phase have a benefit regarding their usability thanks to their low viscosity. In addition, they have another benefit regarding their appearance since they do not show a whitish appearance as emulsion compositions do.

To date, some prior art documents exist relating to sun care compositions having the bi-phase form. For example, JP-A-2015-199689 discloses a composition separated into two phases and containing (A) an oil soluble ultraviolet absorber, (B) a water soluble polymer thickener; and (C) a nonionic surfactant, which can be easily mixed homogenously at the time of use and can be sprayed in a misty state. In addition, JP-A-2016-124817 discloses a double layered cosmetic containing the following components (A)-(D) in an aqueous phase: (A) water-soluble ultraviolet absorber with a sulfonic acid group, (B) a basic substance, (C) water , and (D) a polyhydric alcohol; and the following components (E) and (F) in an oily phase: (E), an oil soluble ultraviolet absorber and (F) a liquid oil.

However, there is still a need for sun care compositions in the bi-phase form, having a better UV protection property and mist-forming quality.

### DISCLOSURE OF INVENTION

An objective of the present invention is to provide a bi-phase sun-care composition having a good UV protecting property and mist-forming quality.

The above objective can be achieved by a bi-phase composition comprising an aqueous phase and an oily phase, wherein the composition comprises:
(a) at least one semi-crystalline or crystalline lipophilic thickener selected from poly C10 -C30 alkyl acrylate and crystalline apolar hydrocarbon-based waxes constituted solely of carbon and hydrogen atoms,
(b) at least one UV filter, and
(c) water, and
wherein the aqueous phase and the oily phase are visually separated at rest.

The viscosity of the oily phase can be more than 5 mPa·s.

The (a) semi-crystalline or crystalline lipophilic thickener may be selected from semi-crystalline polymers.

The (b) UV filter may comprise at least one lipophilic organic UV filter, and may further comprise at least one hydrophilic organic UV filter.

The amount of the semi-crystalline or crystalline lipophilic thickener(s) in the composition may be from 0.001 to 15% by weight, preferably from 0.01 to 5% by weight, and more preferably from 0.05 to 2% by weight, relative to the total weight of the composition.

The composition according to the present invention may comprise at least one oil.

The composition according to the present invention may comprisie cationic, anionic, non-ionic, amphoteric or zwitterionic surfactants or emulsifiers in an amount of 3% by weight or less, preferably 2% by weight or less, and more preferably 1% by weight or less, relative to the total amount of the composition.

The composition according to the present invention may be free of any kind of surfactant or emulsifier.

The composition according to the present invention may be transparent.

The present invention also relates to the composition according to the present invention, for use in a process for protecting keratinous substances, preferably skin, from UV radiation, which is applied to the keratinous substances.

The present invention also relates to a product comprising the composition according to the present invention and a containerselected from a bottle, a tube, a vessel, or a container equipped with a mist-pump, a spray bottle, and a pump bottle.

### BEST MODE FOR CARRYING OUT THE INVENTION

After diligent research, the inventors have discovered that a bi-phase composition comprising semi-crystalline or crystalline lipophilic thickener and UV filter can provide good UV protecting property and mist-forming quality.

Thus, the composition according to the present invention is a bi-phase composition comprising an aqueous phase and an oily phase, wherein the composition comprises:
(a) at least one semi-crystalline or crystalline lipophilic thickener selected from poly C 10 -C30 alkyl acrylate and crystalline apolar hydrocarbon-based waxes constituted solely of carbon and hydrogen atoms,
(b) at least one UV filter, and
(c) water, and
wherein the aqueous phase and the oily phase are visually separated at rest.

Hereinafter, the composition according to the present invention will be explained in a more detailed manner.

### [Composition]

The composition according to the present invention may be intended for use as a cosmetic topical composition. Thus, the composition according to the present invention may be intended for application onto a keratinous substance. Keratinous substance here means a material containing keratin as a main constituent element, and examples thereof include the skin, scalp, nails, lips, hair, and the like. In particular, the composition according to the present invention may be a skin sun care cosmetic composition for protecting skin from UV rays.

The composition according to the present invention has a bi-phase form comprising an aqueous phase and an oily phase.

The bi-phase composition according to the present invention means a composition consisting of two phases of the aqueous phase and the oily phase which are visually and/or macroscopically separated at rest.

The term "aqueous phase" here means a phase that is liquid at room temperature, comprising or consisting of hydrophilic, water-soluble, or water-dispersible substances.

The term "oily phase" here means a phase that is liquid at room temperature, comprising or consisting of lipophilic, oil-soluble, or oil-dispersible substances.

It is preferable that each of the aqueous and oily phases be a continuous phase at rest. Therefore, the bi-phase composition according to the present invention has two distinct layers at rest, each layer consisting of either the aqueous phase or the oily phase. In other words, the aqueous phase and the oily phase are separated at rest by a single interface. This state is clearly different from an emulsion in which one of the phases is dispersed in the other in the form of droplets and thus the phases do not form layers, and are separated by multiple interfaces.

The composition according to the present invention is preferably transparent. The transparency of the composition can be determined by measuring the turbidity with, for example, a turbidimeter (2100Q portable, Hach Company). Preferably, the composition has a turbidity of more than 0, preferably more than 10, and less than 200, preferably less than 150.

The oily phase of the present invention preferably has a viscosity of more than 5 mPa·s, more preferably more than 10 mPa·s at room temperature (25°C). The upper limit of the viscosity of the oily phase is not limited, but in general, the oily phase has a viscosity of less than 1000 mPa·s at room temperature.

The viscosity of the aqueous phase of the present invention is not particularly limited. Preferably, the viscosity of the aqueous phase can range from 15 to 40 Pa·s, more preferably from 17 to 30 Pa·s at room temperature.

For the viscosity measurement, VISCOMAN^{™}(GILSON Technology) can be used. The bulk viscosity (mPaS, PaS) is automatically calculated from the needed aspiration force in the small pipet (10 µL).

The composition according to the present invention is intended for use after mixing the aqueous phase and the oily phase homogenously, for example, by simply shaking it. Since the homogenous composition of the present invention after mixing has a relatively lower viscosity, especially a lower viscosity than an emulsion composition, the present invention has a benefit in that it can be easily sprayed in a misty state without using an aerosol device and easily spread on keratinous substances. Therefore, the composition according to the present invention may be contained in a non-aerosol device, such as a container equipped with a mist generating means, such as a mist-pump, a spray bottle, or a pump bottle.

The pH of the aqueous phase of the present invention is not particularly limited. The pH of the aqueous phase may range from 3.0 to 9.0, preferably from 4.0 to 8.0. The pH of the aqueous phase of the present invention can be adjusted to the desired value using acidifying or basifying agents commonly used in the cosmetics field.

The inventors of the present invention surprisingly found that the mixed composition according to the present invention, in particular applied as mist, can form a homogenous and fine film on keratinous substances, such as skin. Accordingly, the composition can provide improved UV protecting effects.

The amount of the aqueous phase in the composition according to the present invention may range from 20 to 80% by weight, preferably from 30 to 70% by weight, and more preferably from 40 to 60% by weight, relative to the total weight of the composition.

The amount of the oily phase in the composition according to the present invention may range from 20 to 80% by weight, preferably from 30 to 70% by weight, and more preferably from 40 to 60% by weight, relative to the total weight of the composition.

The composition according to the present invention comprises (a) at least one semi-crystalline or crystalline lipophilic thickener, (b) at least one UV filter, and (c) water. These ingredients will be explained in a more detailed manner below.

### (Semi-crystalline or Crystalline Lipophilic Thickener)

The composition according to the present invention comprises (a) at least one semi-crystalline or crystalline lipophilic thickener. Two or more semi-crystalline or crystalline lipophilic thickeners may be used in combination. Thus, a single type of semi-crystalline or crystalline lipophilic thickener or a combination of different types of semi-crystalline or crystalline lipophilic thickeners may be used.

The term "lipophilic" here means a substance which is soluble in oil(s) at a concentration of at least1 % by weight relative to the total weight of the oil(s) at room temperature (25°C) and atmosphere pressure (10⁵ Pa). Therefore, the semi-crystalline or crystalline lipophilic thickener(s) is(are) present in the oily phase of the present invention.

The semi-crystalline or crystalline lipophilic thickener used in the present invention is selected from poly C10-C30 alkyl acrylate and crystalline apolar hydrocarbon-based waxes constituted solely of carbon and hydrogen atoms.

### (I) Semi-crystalline or Crystalline Polymer

The term "semi-crystalline polymer" means polymers comprising a crystallizable portion, a crystallizable pendent and/or end chain or a crystallizable block in the backbone and/or at the ends, and an amorphous portion in the backbone, and having a first-order reversible temperature of change of phase, in particular of melting (solid-liquid transition). When the crystallizable portion is in the form of a crystallizable block of the polymer backbone, the amorphous portion of the polymer is in the form of an amorphous block; the semi-crystalline polymer is, in this case, a block copolymer, for example of the diblock, triblock or multiblock type, comprising at least one crystallizable block and at least one amorphous block. The term "block" generally means at least five identical repeating units. The crystallizable block(s) are then of different chemical nature from the amorphous block(s).

A semi-crystalline polymer has a melting point of greater than or equal to 30°C (especially ranging from 30°C to 80°C), preferably ranging from 30°C to 60°C,and in particular ranging from 40°C to 50°C. This melting point is a first-order temperature of change of state.

This melting point may be measured by any known method and in particular using a differential scanning calorimeter (DSC), for example the calorimeter sold under the name DSC Q2000 by the company TA Instruments.

Advantageously, a tbesemi-crystalline polymer(s) has a number-average molecular mass of greater than or equal to 1000.

Advantageously, a semi-crystalline polymer(s) has a number-average molecular mass Mn ranging from 2000 to 800 000, preferably from 3000 to 500 000, better still from 4000 to 150 000 and especially less than 100 000 and better still from 4000 to 99 000. Preferably, they have a number-average molecular mass of greater than 5600, for example ranging from 5700 to 99 000.

The expression "crystallizable chain or block" means a chain or block which, if it were obtained alone, would change from the amorphous state to the crystalline state reversibly, depending on whether the temperature is above or below the melting point. A "chain" is a group of atoms, which are pendent or lateral relative to the polymer backbone. A "block" is a group of atoms belonging to the backbone, this group constituting one of the repeating units of the polymer. Advantageously, the "pendent crystallizable chain" may be a chain containing at least 6 carbon atoms.

Preferably, the crystallizable block(s) or chain(s) of the semi-crystalline polymers represent at least 30% of the total weight of each polymer and better still at least 40%. The semi-crystalline polymers containing crystallizable blocks are block or multiblock polymers. They may be obtained via polymerization of a monomer containing reactive double bonds (or ethylenic bonds) or via polycondensation. When the polymers are polymers containing crystallizable side chains, these side chains are advantageously in random or statistical form.

Semi-crystalline polymers may be of synthetic origin. Moreover, they do not comprise a polysaccharide backbone. In general, the crystallizable units (chains or blocks) of the semi-crystalline polymers originate from monomer(s) containing crystallizable block(s) or chain(s), used for the manufacture of the semi-crystalline polymers.

Semi-crystalline polymers may be chosen from block copolymers comprising at least one crystallizable block and at least one amorphous block, and homopolymers and copolymers bearing at least one crystallizable side chain per repeating unit, and mixtures thereof.

Examples of semi-crystalline polymers are in particular:
- block copolymers of polyolefins with controlled crystallization, especially those whose monomers are described in EP-A-0 951 897,
- polycondensates, especially of aliphatic or aromatic polyester type or of aliphatic/aromatic copolyester type,
- homopolymers or copolymers bearing at least one crystallizable side chain and homopolymers or copolymers bearing at least one crystallizable block in the backbone, for instance those described in document US-A-5 156 911,
- homopolymers or copolymers bearing at least one crystallizable side chain, in particular containing fluoro group(s), as described in document WO-A-01/19333,
- and mixtures thereof.

In the last two cases, the crystallizable side chain(s) or block(s) are hydrophobic.

### (i) Semi-crystalline polymers containing crystallizable side chains

Mention may be made in particular of those defined in documents US-A-5 156 911 and WO-A-01/19333. They are homopolymers or copolymers comprising from 50% to 100% by weight of units resulting from the polymerization of one or more monomers bearing a crystallizable hydrophobic side chain.

These homopolymers or copolymers are of any nature, provided that they meet the conditions mentioned previously.

They can result:
- from the polymerization, especially the free-radical polymerization, of one or more monomers containing reactive or ethylenic double bond(s) with respect to a polymerization, namely a vinyl, (meth)acrylic or allylic group,
- from the polycondensation of one or more monomers bearing co-reactive groups (carboxylic acid, sulfonic acid, alcohol, amine or isocyanate), such as, for example, polyesters, polyurethanes, polyethers, polyureas or polyamides.

In general, these polymers are chosen especially from homopolymers and copolymers resulting from the polymerization of at least one monomer containing crystallizable chain(s) that may be represented by formula (I): 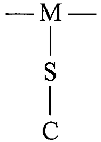 with M representing an atom of the polymer backbone, S representing a spacer and C representing a crystallizable group.

The crystallizable chains "-S-C" may be aliphatic or aromatic, and optionally fluorinated or perfluorinated. "S" especially represents the group (CH₂)ₙ or (CH₂CH₂O)ₙ or (CH₂O), which may be linear or branched or cyclic, with n being an integer ranging from 0 to 22. Preferably, "S" is a linear group. Preferably, "S" and "C" are different.

When the crystallizable chains "-S-C" are hydrocarbon-based aliphatic chains, they comprise hydrocarbon-based alkyl chains containing at least 11 carbon atoms and not more than 40 carbon atoms and better still not more than 24 carbon atoms. They are especially aliphatic chains or alkyl chains containing at least 12 carbon atoms, and they are preferably C₁₄-C₂₄ alkyl chains. When they are fluoroalkyl or perfluoroalkyl chains, they contain at least six fluorinated carbon atoms and especially at least 11 carbon atoms, at least six of which carbon atoms are fluorinated.

As examples of semi-crystalline polymers or copolymers bearing crystallizable chain(s), mention may be made of those resulting from the polymerization of one or more of the following monomers: (meth)acrylates of saturated alkyl with the alkyl group being C₁₄-C₂₄, perfluoroalkyl (meth)acrylates with a C₁₁-C₁₅ perfluoroalkyl group, N-alkyl(meth)acrylamides with the alkyl group being C₁₄ to C₂₄ with or without a fluorine atom, vinyl esters containing alkyl or perfluoro(alkyl) chains with the alkyl group being C₁₄ to C₂₄ (with at least 6 fluorine atoms per perfluoroalkyl chain), vinyl ethers containing alkyl or perfluoro(alkyl) chains with the alkyl group being C₁₄ to C₂₄ and at least 6 fluorine atoms per perfluoroalkyl chain, C₁₄ to C₂₄ alpha-olefins such as, for example, octadecene, para-alkylstyrenes with an alkyl group containing from 12 to 24 carbon atoms, and mixtures thereof.

When the polymers result from a polycondensation, the hydrocarbon-based and/or fluorinated crystallizable chains as defined above are borne by a monomer that may be a diacid, a diol, a diamine or a diisocyanate.

When the polymers are copolymers, they may additionally contain from 0 to 50% of groups Y or Z resulting from the copolymerization:
α) of Y which is a polar or non-polar monomer or a mixture of the two:
When Y is a polar monomer, it is either a monomer bearing polyoxyalkylenated groups (especially oxyethylenated and/or oxypropylenated groups), a hydroxyalkyl (meth)acrylate, for instance hydroxyethyl acrylate, (meth)acrylamide, an N-alkyl(meth)acrylamide, an N,N-dialkyl(meth)acrylamide such as, for example, N,N-diisopropylacrylamide or N-vinylpyrrolidone (NVP), N-vinylcaprolactam, a monomer bearing at least one carboxylic acid group, for instance (meth)acrylic acid, crotonic acid, itaconic acid, maleic acid or fumaric acid, or bearing a carboxylic acid anhydride group, for instance maleic anhydride, and mixtures thereof.

When Y is a non-polar monomer, it may be an ester of the linear, branched or cyclic alkyl (meth)acrylate type, a vinyl ester, an alkyl vinyl ether, an α-olefin, styrene or styrene substituted with a C₁ to C₁₀ alkyl group, for instance α-methylstyrene.

The term "alkyl" means a saturated group especially of C₈ to C₂₄, except where otherwise mentioned, and better still of C₁₄ to C₂₄.

β) of Z which is a polar monomer or a mixture of polar monomers. In this case, Z has the same definition as the "polar Y" defined above.

Preferably, the semi-crystalline polymers containing a crystallizable side chain are alkyl (meth)acrylate or alkyl(meth)acrylamide homopolymers with an alkyl group as defined above, and especially of C₁₄-C₂₄, copolymers of these monomers with a hydrophilic monomer preferably of different nature from (meth)acrylic acid, for instance N-vinylpyrrolidone or hydroxyethyl (meth)acrylate, and mixtures thereof.

### (ii) Polymers bearing in the backbone at least one crystallizable block

These polymers are especially block copolymers consisting of at least two blocks of different chemical nature, one of which is crystallizable.
- The block polymers defined in patent US-A-5 156 911 may be used;
- Block copolymers of olefin or of cycloolefin containing a crystallizable chain, for instance those derived from the block polymerization of:
- cyclobutene, cyclohexene, cyclooctene, norbornene (i.e. bicyclo(2,2,1)-2-heptene), 5-methylnorbomene, 5-ethylnorbomene, 5,6-dimethylnorbomene, 5,5,6-trimethylnorbomene, 5-ethylidenenorbornene, 5-phenylnorbomene, 5-benzylnorbomene, 5-vinylnorbomene, 1,4,5,8-dimethano-1,2,3,4,4a,5,8a-tetrahydronaphthalene, dicyclopenta-diene, or mixtures thereof,
- with ethylene, propylene, 1-butene, 3-methyl-1-butene, 1-hexene, 4-methyl-1-pentene, 1-octene, 1-decene or 1-eicosene, or mixtures thereof.
- and in particular copoly(ethylene/norbomene) blocks and (ethylene/propylene/ethylidene-norbomene) block terpolymers. Those resulting from the block copolymerization of at least two C₂-C₁₆, better still C₂-C₁₂ and even better still C₄-C₁₂ α-olefins such as those mentioned above and in particular block bipolymers of ethylene and of 1-octene may also be used.
- The copolymers may be copolymers containing at least one crystallizable block, the copolymer residue being amorphous (at room temperature). These copolymers may also contain two crystallizable blocks of different-chemical nature. The preferred copolymers are those that simultaneously contain at room temperature a crystallizable block and an amorphous block that are both hydrophobic and lipophilic, sequentially distributed; mention may be made, for example, of polymers containing one of the crystallizable blocks and one of the amorphous blocks below:
- Block that is crystallizable by nature: a) of polyester type, for instance poly(alkylene terephthalate), b) of polyolefin type, for instance polyethylenes or polypropylenes.
- Amorphous and lipophilic block, for instance amorphous polyolefins or copoly(olefin)s such as poly(isobutylene), hydrogenated polybutadiene or hydrogenated poly(isoprene).

As examples of such copolymers containing a crystallizable block and a separate amorphous block, mention may be made of:
α) poly(ε-caprolactone)-b-poly(butadiene) block copolymers, preferably used hydrogenated, such as those described in the article "Melting behaviour of poly(ε-caprolactone)-block-polybutadiene copolymers" from S. Nojima, Macromolecules, 32, 3727-3734 (1999),
β) the hydrogenated block or multiblock poly(butylene terephthalate)-b-poly(isoprene) block copolymers cited in the article "Study of morphological and mechanical properties of PP/PBT" by B. Boutevin et al., Polymer Bulletin, 34, 117-123 (1995),
γ) the poly(ethylene)-b-copoly(ethylene/propylene) block copolymers cited in the articles "Morphology of semicrystalline block copolymers of ethylene-(ethylene-alt-propylene)" by P. Rangarajan et al., Macromolecules, 26, 4640-4645 (1993) and "Polymer aggregates with crystalline cores: the system poly(ethylene)poly(ethylene-propylene)" by P. Richter et al., Macromolecules, 30, 1053-1068 (1997).
δ) the poly(ethylene)-b-poly(ethylethylene) block copolymers mentioned in the general article "Crystallization in block copolymers" by I.W. Hamley, Advances in Polymer Science, vol. 148, 113-137 (1999).

The semicrystalline polymers may or may not be partially crosslinked, provided that the degree of crosslinking does not interfere with their dissolution or dispersion in the liquid fatty phase optionally present in the composition by heating above their melting point. It may then be a case of chemical crosslinking, by reaction with a multifunctional monomer during the polymerization. It may also be a case of physical crosslinking, which may then be due either to the establishment of bonds of hydrogen or dipolar type between groups borne by the polymer, for instance dipolar interactions between carboxylate ionomers, these interactions being in small amount and borne by the polymer backbone; or to a phase separation between the crystallizable blocks and the amorphous blocks, borne by the polymer.

The polymers may be copolymers resulting from the polymerization of at least one monomer containing a crystallizable chain chosen from saturated C₁₄ to C₂₄ alkyl (meth)acrylates, C₁₁ to C₁₅ perfluoroalkyl (meth)acrylates, C₁₄ to C₂₄ N-alkyl(meth)-acrylamides with or without a fluorine atom; vinyl esters containing C₁₄-to C₂₄ alkyl or perfluoroalkyl chains, vinyl ethers containing C₁₄ to C₂₄ alkyl or perfluoroalkyl chains, C₁₄ to C₂₄ alpha-olefins, para-alkylstyrenes with an alkyl group containing from 12 to 24 carbon atoms, with at least one optionally fluorinated C₁ to C₁₀ monocarboxylic acid ester or amide, which may be represented by the following formula (ω): 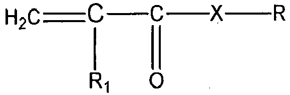 in which R₁ is H or CH₃, R represents an optionally fluorinated C₁-C₁₀ alkyl group and X represents O, NH or NR₂ in which R₂ represents an optionally fluorinated C₁-C₁₀ alkyl group.

The polymers may be derived from a monomer containing a crystallizable chain chosen from saturated C₁₄ to C₂₂ alkyl (meth)acrylates and even more particularly poly(stearyl acrylate) or poly(behenyl acrylate).

The structuring semi-crystalline polymers used in the composition according to the present are polymers having the INCI name "Poly C₁₀-C₃₀ alkyl acrylate", for instance the Intelimer^{®} products from the company Air Products, for instance the product Intelimer^{®} IPA 13-1, which is a polystearyl acrylate of a melting point of 48°C, or the product Intelimer^{®} IPA 13-6, which is a behenyl polymer.

Other examples of semi-crystalline polymers may especially be: those described in Examples 3, 4, 5, 7, 9 and 13 of patent US-A-5 156 911 containing a -COOH group, resulting from the copolymerization of acrylic acid and of C₅ to C₁₆ alkyl (meth)acrylate and more particularly of the copolymerization:
- of acrylic acid, of hexadecyl acrylate and of isodecyl acrylate in a 1/16/3 weight ratio,
- of acrylic acid and of pentadecyl acrylate in a 1/19 weight ratio,
- of acrylic acid, of hexadecyl acrylate and of ethyl acrylate in a 2.5/76.5/20 weight ratio,
- of acrylic acid, of hexadecyl acrylate and of methyl acrylate in a 5/85/10 weight ratio,
- of acrylic acid and of octadecyl methacrylate in a 2.5/97.5 weight ratio,
- of hexadecyl acrylate, of polyethylene glycol methacrylate monomethyl ether containing 8 ethylene glycol units, and of acrylic acid in an 8.5/1/0.5 weight ratio.

Other examples are the structure "O" from National Starch, as described in document US-A-5 736 125, with a melting point of 44°C, and also semi-crystalline polymers with crystallizable pendent chains comprising fluoro groups, as described in Examples 1, 4, 6, 7 and 8 of document WO-A-01/19333.

Other examples are semi-crystalline polymers obtained by copolymerization of stearyl acrylate and of acrylic acid or NVP as described in document US-A-5 519 063 or EP-A-550 745, with melting points of 40°C and 38°C, respectively.

Other examples are semi-crystalline polymers obtained by copolymerization of behenyl acrylate and of acrylic acid or NVP, as described in documents US-A-5 519 063 and EP-A-550 745, with melting points of 60°C and 58°C, respectively.

Finally, the semi-crystalline polymers may be waxy polymers obtained by metallocene catalysis, such as those described in patent application US 2007/0 031 361.

These polymers are homopolymers or copolymers of ethylene and/or propylene prepared via metallocene catalysis, i.e. by polymerization at low pressure and in the presence of a metallocene catalyst.

The weight-average molecular mass (Mw) of the waxes obtained via metallocene catalysis described in that document is less than or equal to 25 000 g/mol and ranges, for example, from 2000 to 22 000 g/mol and better still from 4000 to 20 000 g/mol.

The number-average molecular mass (Mn) of the waxes obtained via metallocene catalysis described in that document is preferably less than or equal to 15 000 g/mol and ranges, for example, from 1000 to 12 000 g/mol and better still from 2000 to 10 000 g/mol.

The polydispersity index I of the polymer is equal to the ratio of the weight-average molecular mass Mw to the number-average molecular mass Mn. Preferably, the polydispersity index of the waxy polymers is between 1.5 and 10, moer preferably between 1.5 and 5, even more preferably between 1.5 and 3 and better still between 2 and 2.5.

The waxy homopolymers and copolymers may be obtained in a known manner from ethylene and/or propylene monomers, for example via metallocene catalysis according to the process described in document EP 571 882.

The homopolymers and copolymers of ethylene and/or propylene prepared via metallocene catalysis may be unmodified or "polar"-modified (polar-modified waxes, i.e. waxes modified such that they have the properties of a polar wax). The polar-modified waxy homopolymers and copolymers may be prepared in a known manner from unmodified waxy homopolymers and copolymers such as those described previously by oxidation with gases containing oxygen, such as air, or by grafting with polar monomers such as maleic acid or acrylic acid or alternatively derivatives of these acids. These two routes enabling polar modification of the polyolefins obtained via metallocene catalysis are described, respectively, in documents EP 890 583 and US 5 998 547, for example, the content of these two documents being incorporated herein by reference.

The polar-modified homopolymers and copolymers of ethylene and/or propylene prepared via metallocene catalysis may be modified such that they have hydrophilic properties. Examples that may be mentioned include ethylene and/or propylene homopolymers or copolymers modified by the presence of hydrophilic groups such as maleic anhydride, acrylate, methacrylate, polyvinylpyrrolidone (PVP), etc.

Waxy ethylene and/or propylene homopolymers or copolymers modified by the presence of hydrophilic groups such as maleic anhydride or acrylate are an example,

Examples that may be mentioned include:
- polypropylene waxes modified with maleic anhydride (PPMA) sold by the company Clariant, or polypropylene-ethylene-maleic anhydride copolymers, such as those sold by the company Clariant under thename LicoCare, for instance LicoCare.PP207 LP3349, LicoCare CM401 LP3345, LicoCare CA301 LP3346 and LicoCare CA302 LP3347 or alternatively
- the unmodified polyethylene waxes sold by the company Clariant, such as the product LicoCare PE 102 LP3329.

### (II) Semi-crystalline or Crystalline Wax

Semi-crystalline or crystalline waxes are chosen from polar and apolar hydrocarbon-based waxes, or mixtures thereof.

The term "wax(es)", under consideration in the context of the present invention are generally lipophilic compounds that are solid at room temperature (25°C), with a solid/liquid reversible change of state, having a melting point of greater than or equal to 30°C, which may be up to 200°C and especially up to 120°C.

In particular, the semi-crystalline or crystalline waxes may have a melting point ot greater than or equal to 40°C, preterably greater than or equal to 50°C, and in particular greater than or equal to 60°C. Furthermore, the semi-crystalline or crystalline waxes may have a melting point of less than or equal to 100°C, preferably less than or equal to 85°C, and especially less than or equal to 70°C

For the purposes of the present invention, the melting point corresponds to the temperature of the most endothermic peak observed in thermal analysis (DSC) as described in the standard ISO 11357-3; 1999. The melting point of the wax may be measured using a differential scanning calorimeter (DSC), for example the calorimeter sold under the name DSC Q2000 by the company TA Instruments.

The semi-crystalline or crystalline waxes comprise at least one crystallizable part, which can be visible by X-ray observation.

The exemplified measurement protocol of the melting point is as follows:
A sample of 5 mg of wax placed in a crucible is subjected to a first temperature rise ranging from -20°C to 120°C, at a heating rate of 10°C/minute, it is then cooled from 120°C to -20°C at a cooling rate of 10°C/minute and is finally subjected to a second temperature increase ranging from -20°C to 120°C at a heating rate of 5°C/minute. During the second temperature increase, the following parameters are measured. The melting point (Mp) of the wax, as mentioned previously, corresponds to the temperature of the most endothermic peak of the melting curve observed, and represents the variation of the difference in power absorbed as a function of the temperature.

A semi-crystalline or crystalline wax may have a molecular weight between 100 and 3,000 Dalton, preferable between 200 and 2,000 Dalton, and more preferably between 300 and 1,000 Dalton.

The waxes used in the present invention are crystalline apolar waxes.

### (i) Apolar wax

For the purposes of the present invention, the term *"apolar wax"* means a wax whose solubility parameter at 25°C as defined below, δₐ, is equal to 0 (J/cm³)^{½}.

Apolar waxes according to the present invention are hydrocarbon-based waxes constituted solely of carbon and hydrogen atoms, and free of heteroatoms such as N, O, Si and P.

The term "hydrocarbon-based wax" means a wax formed essentially from, or even constituted of, carbon and hydrogen atoms, and optionally oxygen and nitrogen atoms, and not containing any silicon or fluorine atoms. It may contain alcohol, ester, ether, carboxylic acid, amine and/or amide groups.

The definition and calculation of the solubility parameters in the Hansen three-dimensional solubility space are described in the article by C.M. Hansen: The three-dimensional solubility parameters, J. Paint Technol. 39, 105 (1967).

According to this Hansen space:
- δ_{D} characterizes the London dispersion forces derived from the formation of dipoles induced during molecular impacts;
- δₚ characterizes the Debye interaction forces between permanent dipoles and also the Keesom interaction forces between induced dipoles and permanent dipoles;
- δₕ characterizes the specific interaction forces (such as hydrogen bonding, acid/base, donor/acceptor, etc.); and
- δₐ is determined by the equation: δₐ= (δₚ² + δₕ²)^{½}

The parameters δₚ, δₕ, δ_{D} and δₐ are expressed in (J/cm³)^{½}.

More particularly, the apolar wax may be chosen from microcrystalline waxes, paraffin waxes, ozokerite, polyethylene waxes, polymethylene waxes and microwaxes, and mixtures thereof.

As microcrystalline waxes that may be used, mention may be made of Multiwax W 445^{®} sold by the company Sonneborn, and Microwax HW^{®} and Base Wax 30540^{®} sold by the company Paramelt.

An ozokerite that may be mentioned is Ozokerite Wax SP 1020 P.

Polyethylene waxes that may be mentioned include Performalene 500-L Polyethylene and Performalene 400 Polyethylene sold by New Phase Technologies.

Polymethylene waxes that may be mentioned include the Polymethylene Wax sold under the reference Cirebelle 303, which has a melting point of 61°C to 67°C; and the Polymethylene Wax sold under the reference Cirebelle 108, which has a melting point of 79°C to 84°C, sold by Cirebelle.

As microwaxes that may be used in the compositions according to the present invention as apolar wax, mention may be made especially of polyethylene microwaxes such as those sold under the names Micropoly 200^{®}, 220^{®}, 220L^{®} and 250S^{®} by the company Micro Powders.

### (ii) Polar wax

For the purposes of the present invention, the term *"polar wax"* means a wax whose solubility parameter at 25°C, δₐ, is other than 0 (J/cm³)^{½}.

The term *"polar wax"* here means a wax whose chemical structure is formed essentially from, or even constituted of, carbon and hydrogen atoms, and comprising at least one highly electronegative heteroatom such as an oxygen, nitrogen, silicon or phosphorus atom.

As the hydrocarbon-based polar wax, a wax chosen from ester waxes may be an example.

The term *"hydrocarbon-based"* means a compound formed essentially from, or even constituted of, carbon and hydrogen atoms, and optionally oxygen and nitrogen atoms, and not containing any silicon or fluorine atoms.

According to the present invention, the term *"ester wax"* means a wax comprising at least one ester function.

The following are examples of an ester wax:
i) waxes of formula R₁COOR₂ in which R₁ and R₂ represent linear, branched or cyclic aliphatic chains in which the number of atoms ranges from 10 to 50, which may contain a heteroatom such as O, N or P and whose melting point ranges from 25 to 120°C.

Particular examples of an ester wax is C₂₀-C₄₀ alkyl (hydroxystearyloxy)stearate (the alkyl group comprising from 20 to 40 carbon atoms), alone or as a mixture, or a C₂₀-C₄₀ alkyl stearate. Such waxes are especially sold under the names Kester Wax K 82 P^{®}, Hydroxypolyester K 82 P^{®}, Kester Wax K 80 P^{®} and Kester Wax K82H by the company Koster Keunen.
ii) glycol and butylene glycol montanate (octacosanoate) waxes such as the wax Licowax KPS Flakes (INCI name: glycol montanate) sold by the company Clariant.
iii) bis(1,1,1-trimethylolpropane) tetrastearate, sold under the name Hest 2T-4S^{®} by the company Heterene.
iv) diester waxes of a dicarboxylic acid of general formula R³-(-OCO-R⁴-COO-R⁵), in which R³ and R⁵ are identical or different, preferably identical and represent a C₄-C₃₀ alkyl group (alkyl group comprising from 4 to 30 carbon atoms) and R⁴ represents a linear or branched C₄-C₃₀ aliphatic group (alkyl group comprising from 4 to 30 carbon atoms) which may or may not contain one or more unsaturated groups, and preferably that is linear and unsaturated.
v) Mention may also be made of the waxes obtained by catalytic hydrogenation of animal or plant oils having linear or branched C₈-C₃₂ fatty chains, for example such as hydrogenated jojoba oil, hydrogenated sunflower oil, hydrogenated castor oil, hydrogenated coconut oil, and also the waxes obtained by hydrogenation of castor oil esterified with cetyl alcohol, such as those sold under the names Phytowax Ricin 16L64^{®} and 22L73^{®} by the company Sophim. Such waxes are described in patent application FR-A-2792190 and the waxes obtained by hydrogenation of olive oil esterified with stearyl alcohol such as that sold under the name Phytowax Olive 18 L 57, or the like.
vi) beeswax, synthetic beeswax, polyglycerolated beeswax, carnauba wax, candelilla wax, oxypropylenated lanolin wax, rice bran wax, ouricury wax, esparto grass wax, cork fibre wax, sugar cane wax, Japan wax, sumach wax, montan wax, orange wax, laurel wax and hydrogenated jojoba wax. Candelilla wax is preferably used.

According to the present invention, the wax is a crystalline apolar hydrocarbon-based wax constituted solely of carbon and hydrogen atoms, such as a polymethylene wax.

The amount of the semi-crystalline or crystalline lipophilic thickener(s) in the composition according to the present invention may be 0.001% by weight or more, preferably 0.01% by weight or more, and more preferably 0.05% by weight or more, relative to the total weight of the composition.

The amount of the semi-crystalline or crystalline lipophilic thickener(s) in the composition according to the present invention may be 15% by weight or less, preferably 5% by weight or less, and more preferably 2% by weight or less, relative to the total weight of the composition. Specifically, the amount of the semi-crystalline or crystalline lipophilic thickener(s) in the composition according to the present invention may be from 0.001 to 15% by weight, preferably from 0.01 to 5% by weight, and more preferably from 0.05 to 2% by weight, relative to the total weight of the composition.

### (UV Filter)

The composition according to the present invention comprises (b) at least one UV filter. Two or more UV filters may be used in combination. Thus, a single type of UV filter or a combination of different types of UV filters may be used.

There is no limitation to the type of UV filter. The UV filter can be selected from organic UV filters, inorganic UV filters, and mixtures thereof.

The term "UV" here means ultraviolet radiation and comprises the UV-B region (260-320 nm in wavelength) and the UV-A region (320-400 nm in wavelength). Therefore, a UV filter means any material which has filtering effects on ultraviolet radiation in wavelengths of the UV-A and/or UV-B regions.

The UV filter(s) used for the present invention may be active in the UV-A and/or UV-B region, preferably in both of the UV-A and UV-B regions alone or in combination. Therefore, the UV filter(s) used in the present invention include(s) a UV-A filter capable of absorbing UV radiation from 320 to 400 nm, a UV-B filter capable of absorbing UV radiation from 280 to 320 nm, and a UV-A and UV-B filter capable of absorbing UV radiation from 280 to 400 nm.

### (I) Organic UV Filter

The organic UV filters used for the present invention can be lipophilic organic UV filters and/or hydrophilic organic UV filters. Preferably, the composition according to the present invention comprises at least one lipophilic organic UV filter, and more preferably comprises a combination of at least one lipophilic organic UV filter and at least one hydrophilic organic UV filter.

### (i) Lipophilic organic UV filter

The term "lipophilic organic UV filter" here means organic UV filters which are soluble in oils at a concentration of at least 1 % by weight relative to the total weight of the oils at room temperature (25°C) and atmosphere pressure (10⁵ Pa). Therefore, the lipophilic organic UV filter(s) is(are) present in the oily phase of the present invention.

The lipophilic organic UV-A filters used in the present invention may include, but are not limited to, aminobenzophenone compounds, dibenzoylmethane compounds, anthranilic acid compounds, and 4,4-diarylbutadiene compounds.

As the aminobenzophenone compounds, mention may be made of n-hexyl 2-(4-diethlamino-2-hydroxybenzoyl)benzoate, the alternative name of which is diethylamino hydroxybenzoyl hexyl benzoate (DHHB), sold under the trade name "Uvinul A+" from BASF.

As the dibenzoylmethane compounds, mention may be made of 4-isopropyldibenzoylmethane, sold under the name of "Eusolex 8020" from Merck, 1-(4-methoxy-1-benzofuran-5-yl)-3-phenylpropane-1,3-dione, sold under the name of "Pongamol" from Quest, 1-(4-(tert-butyl)phenyl)-3-(2-hydroxyphenyl)propane-1,3-dione, and butyl methoxydibenzoylmethane, sold under the trade name "Parsol 1789" from Hoffmann-La Roche.

As the anthranilic acid compounds, mention may be made of menthyl anthranilate marketed under the name "NEO HELIPAN MA" by Symrise.

As the 4,4-diarylbutadiene compounds, mention may be made of 1,1 -dicarboxy (2,2'-dimethylpropyl)-4,4-diphenylbutadiene and diphenyl butadiene malonates and malononitriles.

The lipophilic organic UV-B filters used in the present invention may include, but are not limited to, triazine compounds, para-aminobenzoic acid compounds, salicylic compounds, cinnamate compounds, β,β-diphenylacrylate compounds, benzylidenecamphor compounds, phenylbenzimidazole compounds, imidazoline compounds, benzalmalonate compounds, and mecocyanine compounds.

As the triazine compounds, mention may be made of ethylhexyl triazone, marketed under the name "UVINUL T-150" by BASF, diethylhexyl butamido triazone, marketed under the name "UVASORB HEB" by SIGMA 2V, 2,4,6-tris(dineopentyl 4'-aminobenzalmalonate)-s-triazine, 2,4,6-tris(diisobutyl 4'-aminobenzalmalonate)-s-triazine, 2,4-bis(dineopentyl 4'-aminobenzalmalonate)-6-(n-butyl 4'-aminobenzoate)-s-triazine, 2,4-bis(n-butyl 4'-aminobenzoate)-6-(aminopropyltrisiloxane)-s-triazine.

As the para-aminobenzoic acid derivatives, mention may be made of para-aminobenzoates (PABA), for example, ethyl PABA (para-aminobenzoate), ethyl dihydroxypropyl PABA, and ethylhexyl dimethyl PABA, marketed under the name "ESCALOL 5972 from ISP.

As the salicylic compounds, mention may be made of homosalate, marketed under the name "Eusolex HMS" by Rona/EM industries, and ethylhexyl salicylate, marketed under the name "NEO HELIOPAN OS" by Symrise.

As the cinnamate compounds, mention may be made of ethylhexyl methoxycinnamate, marketed under the name "PARSOL CX" by DSM NUTRITIONAL PRODUCTS, isopropyl ethoxy cinnamate, isoamyl methoxy cinnamate, marketed under the name "NEO HELIOPAN E 1000" by Symrise, diisopropyl methylcinnamate, cinoxate, and glyceryl ethylhexanoate dimethoxycinnamate.

As the β,β-diphenylacrylate compounds, mention may be made of octocrylene, marketed under the name "UVINUL N539" by BASF, and etocrylene, marketed under the name "UVINUL N35" by BASF.

As the benzylidenecamphor compounds, mention may be made of 3-benzylidene camphor, marketed under the name "MEXORYL SD" from CHIMEX, methylbenzylidene camphor, marketed under the name "EUSOLEX 6300" by MERCK, polyacrylamidomethyl benzylidene Camphor, marketed under the name "MEXORYL SW" by CHIMEX, and terephthalylidene dicamphor sulfonic acid, marketed under the name "Mexoryl SX" by Chimex.

As the phenylbenzimidazole compounds, mention may be made of phenylbenzimidazole sulfonic acid, marketed under the name "Eusolex 232" by Merck, and disodium phenyl dibenzimidazole tetrasulfonate, marketed under the name "Neo Heliopan AP" by Haarmann and Reimer.

As the imidazoline compounds, mention may be made of ethylhexyl dimethoxybenzylidene dioxoimidazoline propionate.

As the benzalmalonate compounds, mention may be made of polyorganosiloxane containing a benzalmalonate moiety, for example, Polysilicone-15, marketed under the name "Parsol SLX" by DSM NUTRITIONAL PRODUCTS, and di-neopentyl 4'-methoxybenzalmalonate.

The lipophilic organic UV filters of the present invention may comprise lipophilic UV-A and UV-B filters. The following are non-limited examples of the lipophilic organic UV-A and UV-B filters:
- Benzophenone compounds, such as benzophenone-1 marketed underthe name "UVINUL 400" by BASF, benzophenone-2 marketed under the name "UVINUL 500" by BASF, benzophenone-3 or oxybenzone marketed under the name "UVINUL M40" by BASF, benzophenone-6 marketed under the name "Helisorb 11" by Norquay, benzophenone-8 marketed under the name "Spectra-Sorb UV-24" by American Cyanamid, benzophenone-10, benzophenone-11, and benzophenone-12;
- benzotriazole compounds such as drometrizole trisiloxane marketed under the name "Silatrizole" by Rhodia Chimie and bumetrizole marketed under the name "TINOGUARTD AS" by CIBA-GEIGY;
- bis-resorcinyl triazine compounds, such as bis-ethylhexyloxyphenol methoxyphenyl triazine marketed under the name "TINOSORB S" by CIBA-GEIGY; and
- benzoxazole compounds, such as 2,4-bis[5-(1-dimethylpropyl) benzoxazol-2-yl(4-phenyl)imino]-6-(2-ethylhexyl)imino-1,3,5-triazine marketed under the name "Uvasorb K2A" by Sigma 3V.

Preferably, the lipophilic organic UV filter of the present invention is selected from a combination of at least one lipophilic organic UV-A filter and at least one lipophilic organic UV-B filter. Preferably, the lipophilic organic UV filter may be selected from dibenzoylmethane compounds, triazine compounds, salicylic compounds, β,β-diphenylacrylate compounds, bis-resorcinyl triazine compounds, and mixtures thereof.

In one preferred embodiment of the present invention, the amount of the lipophilic organic UV filter(s) in the composition according to the present invention is at least 1% by weight, preferably at least 5% by weight, and more preferably at least 20% by weight, and is less than or equal to 50% by weight, preferably less than or equal to 40% by weight, and more preferably less than or equal to 35% by weight, relative to the total weight of the composition.

In one particular embodiment of the present invention, the amount of the lipophilic organic UV filter(s) in the oily phase of the present invention is at least 30% by weight, preferably at least 40% by weight, and more preferably at least 50% by weight, and is less than or equal to 80% by weight, preferably less than or equal to 70% by weight, and more preferably less than or equal to 60% by weight, relative to the total weight of the oily phase.

### (ii) Hydrophilic organic UV filter

The term "hydrophilic organic UV filter" here means organic UV filters which are soluble in water at a concentration of at least 1 % by weight relative to the total weight of the water at room temperature (25°C) and atmosphere pressure (10⁵ Pa). Therefore, the hydrophilic organic UV filter(s) is(are) present in the aqueous phase of the present invention.

The hydrophilic organic UV-A filter includes, but is not limited to:
Terephthalylidene Dicamphor Sulfonic Acid and salts thereof, such as that manufactured under the name "Mexoryl SX" by Chimex,
Bisbenzoxazolyl derivatives, such as described in Patent, EP 669 323 and US 2,463,264, more particularly the compound Disodium Phenyl Dibenzimidazole Tetrasulfonate and salts thereof, sold under the trade name "Neo Heliopan AP" by Haarmann and Reimer.

The hydrophilic organic UV-B filter includes, but is not limited to:
p-Aminobenzoic (PABA) derivatives, such as PABA, Glyceryl PABA and PEG-25 PABA,
and salts thereof such as sold under the name "Uvinul P25" by BASF,
Phenylbenzimidazole Sulfonic Acid and salts thereof, such as sold in particular under the trade name "Eusolex 232" by Merck,
Ferulic Acid and salts thereof,
Salicylic Acid and salts thereof,
DEA methoxycinnamate and salts thereof,
Benzylidene Camphor Sulfonic Acid and salts thereof, such as that manufactured under the name "Mexoryl SL" by Chimex,
Camphor Benzalkonium Methosulfate and salts thereof, such as that manufactured under the name "Mexoryl SO" by Chimex.

The hydrophilic organic UV-A and UV-B filter includes, but is not limited to:
Benzophenone-4 and salts thereof, such as those sold under the trade name "Uvinul MS40" by BASF,
Benzophenone-5 and salts thereof, and
Benzophenone-9 and salts thereof.

The salts of the compounds that may be used are chosen in particular from salts of alkali metals, for example sodium or potassium; salts of alkaline-earth metals, for example calcium, magnesium or strontium; metal salts, for example zinc, aluminum, manganese or copper; salts of ammonium of formula NH⁴⁺; quaternary ammonium salts; salts of organic amines, for instance salts of methylamine, dimethylamine, trimethylamine, triethylamine, ethylamine, 2-hydroxyethylamine, bis(2-hydroxyethyl)amine or tris(2-hydroxyethyl)amine; lysine or arginine salts. Salts chosen from sodium, potassium, magnesium, strontium, copper, manganese or zinc salts are preferably used. The sodium salt is preferentially used.

Preferably, the hydrophilic organic UV filter of the present invention is selected from a combination of at least one hydrophilic organic UV-A filter and at least one hydrophilic organic UV-B filter. In particular, the hydrophilic organic UV filter is a combination of phenylbenzimidazole sulfonic acid and terephthalylidene dicamphor sulfonic acid.

In one preferred embodiment of the present invention, the amount of the hydrophilic organic UV filter(s) in the composition according to the present invention is at least 0.1% by weight, preferably at least 0.5% by weight, and more preferably at least 1% by weight, and is less than or equal to 15% by weight, preferably less than or equal to 10% by weight, and more preferably less than or equal to 5% by weight, relative to the total weight of the composition.

In one particular embodiment of the present invention, the amount of the hydrophilic organic UV filter(s) in the aqueous phase of the present invention is at least 0.5% by weight, preferably at least 1% by weight, and more preferably at least 3% by weight, and is less than or equal to 30% by weight, preferably less than or equal to 20% by weight, and more preferably less than or equal to 10% by weight, relative to the total weight of the aqueous phase.

### (II) Inorganic UV filter

The inorganic UV filter may be hydrophilic and/or lipophilic. The inorganic UV filter is preferably insoluble in solvents such as water and ethanol commonly used in cosmetics.

It is preferable that the inorganic UV filter be in the form of a fine particle such that the mean (primary) particle diameter thereof ranges from 1 nm to 50 µm, preferably 5 nm to 500 nm, and more preferably 10 nm to 200 nm. The mean (primary) particle size or mean (primary) particle diameter here is an arithmetic mean diameter.

The inorganic UV filter can be selected from the group consisting of metal oxides which may or may not be coated, and mixtures thereof.

Preferably, the inorganic UV filter is selected from pigments (mean size of the primary particles: generally from 5 nm to 50 nm, preferably from 10 nm to 50 nm) formed of metal oxides, such as, for example, pigments formed of titanium oxide (amorphous or crystalline in the rutile and/or anatase form), iron oxide, zinc oxide, zirconium oxide, or cerium oxide, which are all UV photoprotective agents that are well known per se. Preferably, the inorganic UV filter is selected from titanium oxide, zinc oxide, and more preferably titanium oxide.

The inorganic UV filter may or may not be coated. The inorganic UV filter may have at least one coating. The coating may comprise at least one compound selected from the group consisting of alumina, silica, aluminum hydroxide, silicones, silanes, fatty acids or salts thereof (such as sodium, potassium, zinc, iron, or aluminum salts), fatty alcohols, lecithin, amino acids, polysaccharides, proteins, alkanolamines; waxes such as beeswax, (meth)acrylic polymers, organic UV filters, and (per)fluoro compounds.

It is preferable for the coating to include at least one organic UV filter. As the organic UV filter in the coating, a dibenzoylmethane derivative such as butyl methoxydibenzoylmethane (Avobenzone) and 2,2'-Methylenebis[6-(2H-Benzotriazol-2-yl)-4-(1,1,3,3-Tetramethyl-Butyl) Phenol] (Methylene Bis-Benzotriazolyl Tetramethylbutylphenol) marketed as "TINOSORB M" by BASF may be preferable.

In a known manner, the silicones in the coating(s) may be organosilicon polymers or oligomers comprising a linear or cyclic and branched or cross-linked structure, of variable molecular weight, obtained by polymerization and/or polycondensation of suitable functional silanes and essentially composed of repeated main units in which the silicon atoms are connected to one another via oxygen atoms (siloxane bond), optionally substituted hydrocarbon radicals being connected directly to said silicon atoms via a carbon atom.

The term "silicones" also encompasses silanes necessary for their preparation, in particular alkylsilanes.

The silicones used for the coating(s) can preferably be selected from the group consisting of alkylsilanes, polydialkylsiloxanes, and polyalkylhydrosiloxanes. More preferably still, the silicones are selected from the group consisting of octyltrimethylsilane, polydimethylsiloxanes, and polymethylhydrosiloxanes.

Of course, the inorganic UV filters made of metal oxides may, before their treatment with silicones, have been treated with other surfacing agents, in particular with cerium oxide, alumina, silica, aluminum compounds, silicon compounds, or their mixtures.

The coated inorganic UV filter may have been prepared by subjecting the inorganic UV filter to one or more surface treatments of a chemical, electronic, mechanochemical, and/or mechanical nature with any of the compounds as described above, as well as polyethylenes, metal alkoxides (titanium or aluminum alkoxides), metal oxides, sodium hexametaphosphate, and those shown, for example, in Cosmetics & Toiletries, February 1990, Vol. 105, pp. 53-64.

The coated inorganic UV filters may be titanium oxides coated:
with silica, such as the product "Sunveil" from Ikeda, and "Sunsil TIN 50" from Sunjin Chemical;
with silica and with iron oxide, such as the product "Sunveil F" from Ikeda;
with silica and with alumina, such as the products "Microtitanium Dioxide MT 500 SA" from Tayca, "Tioveil" from Tioxide, and "Mirasun TiW 60" from Rhodia;
with alumina, such as the products "Tipaque TTO-55 (B)" and "Tipaque TTO-55 (A)" from Ishihara, and "UVT 14/4" from Kemira;
with alumina and with aluminum stearate, such as the product "Microtitanium Dioxide MT 100 T, MT 100 TX, MT 100 Z or MT-01" from Tayca, the products "Solaveil CT-10 W" and "Solaveil CT 100" from Uniquema, and the product "Eusolex T-AVO" from Merck;
with alumina and with aluminum laurate, such as the product "Microtitanium Dioxide MT 100 S" from Tayca;
with iron oxide and with iron stearate, such as the product "Microtitanium Dioxide MT 100 F" from Tayca;
with zinc oxide and with zinc stearate, such as the product "BR351" from Tayca;
with silica and with alumina and treated-with a silicone, such as the products "Microtitanium Dioxide MT 600 SAS", "Microtitanium Dioxide MT 500 SAS", and "Microtitanium Dioxide MT 100 SAS" from Tayca;
with silica, with alumina and with aluminum stearate and treated with a silicone, such as the product "STT-30-DS" from Titan Kogyo;
with silica and treated with a silicone, such as the product "UV-Titan X 195" from Kemira;
with alumina and treated with a silicone, such as the products "Tipaque TTO-55 (S)" from Ishihara or "UV Titan M 262" from Kemira;
with triethanolamine, such as the product "STT-65-S" from Titan Kogyo;
with stearic acid, such as the product "Tipaque TTO-55 (C)" from Ishihara; or
with sodium hexametaphosphate, such as the product "Microtitanium Dioxide MT 150 W" from Tayca.

Other titanium oxide pigments treated with a silicone are preferably TiO₂ treated with octyltrimethylsilane for which the mean size of the individual particles is from 25 and 40 nm, such as that marketed under the trademark "T 805" by Degussa Silices, TiO₂ treated with a polydimethylsiloxane for which the mean size of the individual particles is 21 nm, such as that marketed under the trademark "70250 Cardre UF TiO₂Si₃" by Cardre, and anatase/rutile TiO₂ treated with a polydimethylhydrosiloxane for which the mean size of the individual particles is 25 nm, such as that marketed under the trademark "Microtitanium Dioxide USP Grade Hydrophobic" by Color Techniques.

Preferably, the following coated TiO₂ can be used as the coated inorganic UV filter:
Stearic acid (and) Aluminum Hydroxide (and) TiO₂, such as the product "MT-100 TV" from Tayca, with a mean primary particle diameter of 15 nm;
Dimethicone (and) Stearic Acid (and) Aluminum Hydroxide (and) TiO₂, such as the product "SA-TTO-S4" from Miyoshi Kasei, with a mean primary particle diameter of 15 nm;
Silica (and) TiO₂, such as the product "MT-100 WP" from Tayca, with a mean primary particle diameter of 15 nm;
Dimethicone (and) Silica (and) Aluminum Hydroxide (and) TiO₂, such as the product "MT-Y02" and "MT-Y-110 M3S" from Tayca, with a mean primary particle diameter of 10 nm;
Dimethicone (and) Aluminum Hydroxide (and) TiO₂, such as the product "SA-TTO-S3" from Miyoshi Kasei, with a mean primary particle diameter of 15 nm;
Dimethicone (and) Alumina (and) TiO₂, such as the product "UV TITAN M170" from Sachtleben, with a mean primary particle diameter of 15 nm; and
Silica (and) Aluminum Hydroxide (and) Alginic Acid (and) TiO₂, such as the product "MT-100 AQ" from Tayca, with a mean primary particle diameter of 15 nm.

In terms of UV filtering ability, TiO₂ coated with at least one organic UV filter is more preferable. For example, Avobenzone (and) Stearic Acid (and) Aluminum Hydroxide (and) TiO₂, such as the product "HXMT-100ZA" from Tayca, with a mean primary particle diameter of 15 nm, can be used.

The uncoated titanium oxide pigments are, for example, marketed by Tayca under the trademarks "Microtitanium Dioxide MT500B" or "Microtitanium Dioxide MT600B", by Degussa under the trademark "P 25", by Wacker under the trademark "Oxyde de titane transparent PW", by Miyoshi Kasei under the trademark "UFTR", by Tomen under the trademark "ITS" and by Tioxide under the trademark "Tioveil AQ".

The uncoated zinc oxide pigments are, for example:
those marketed under the trademark "Z-cote" by Sunsmart;
those marketed under the trademark "Nanox" by Elementis; and
those marketed under the trademark "Nanogard WCD 2025" by Nanophase Technologies.

The coated zinc oxide pigments are, for example:
those marketed under the trademark "Oxide Zinc CS-5" by Toshiba (ZnO coated with polymethylhydrosiloxane);
those marketed under the trademark "Nanogard Zinc Oxide FN" by Nanophase Technologies (as a 40% dispersion in Finsolv TN, C₁₂-C₁₅ alkyl benzoate);
those marketed under the trademark "Daitopersion Zn-30" and "Daitopersion Zn-50" by Daito (dispersions in oxyethylenated polydimethylsiloxane/cyclopolymethylsiloxane comprising 30% or 50% of zinc nano-oxides coated with silica and polymethylhydrosiloxane);
those marketed under the trademark "NFD Ultrafine ZnO" by Daikin (ZnO coated with phosphate of perfluoroalkyl and a copolymer based on perfluoroalkylethyl as a dispersion in cyclopentasiloxane);
those marketed under the trademark "SPD-Z1" by Shin-Etsu (ZnO coated with a silicone-grafted acrylic polymer dispersed in cyclodimethylsiloxane);
those marketed under the trademark "Escalol Z100" by ISP (alumina-treated ZnO dispersed in an ethylhexyl methoxycinnamate/PVP-hexadecene copolymer/methicone mixture);
those marketed under the trademark "Fuji ZnO-SMS-10" by Fuji Pigment (ZnO coated with silica and polymethylsilsesquioxane); and those marketed under the trademark "Nanox Gel TN" by Elementis (ZnO dispersed at 55% in C₁₂-C₁₅ alkyl benzoate with hydroxystearic acid polycondensate).

The uncoated cerium oxide pigments are marketed, for example, under the trademark "Colloidal Cerium Oxide" by Rhone-Poulenc.

The uncoated iron oxide pigments are, for example, marketed by Arnaud under the trademarks "Nanogard WCD 2002 (FE 45B)", "Nanogard Iron FE 45 BL AQ", "Nanogard FE 45R AQ", and "Nanogard WCD 2006 (FE 45R)", or by Mitsubishi under the trademark "TY-220".

The coated iron oxide pigments are, for example, marketed by Arnaud under the trademarks "Nanogard WCD 2008 (FE 45B FN)", "Nanogard WCD 2009 (FE 45B 556)", "Nanogard FE 45 BL 345", and "Nanogard FE 45 BL", or by BASF under the trademark "Oxyde de fer transparent".

Mention may also be made of mixtures of metal oxides, in particular of titanium dioxide and of cerium dioxide, including a mixture of equal weights of titanium dioxide coated with silica and of cerium dioxide coated with silica marketed by Ikeda under the trademark "Sunveil A", and also a mixture of titanium dioxide and of zinc dioxide coated with alumina, with silica and with silicone, such as the product "M 261" marketed by Kemira, or coated with alumina, with silica and with glycerol, such as the product "M 211" marketed by Kemira.

Coated inorganic UV filters are preferable, because the UV filtering effects of the inorganic UV filters can be enhanced. In addition, the coating(s) may help uniformly or homogeneously disperse the UV filters in the composition according to the present invention.

The amount of the UV filter(s)_in the composition according to the present invention may be 5% by weight or more, preferably 10% by weight or more, more preferably 20% by weight or more, and even more preferably 30% by weight or more, relative to the total weight of the composition.

The amount of the UV filter(s) in the composition according to the present invention may be 60% by weight or less, preferably 50% by weight or less, and more preferably 40% by weight or less, relative to the total weight of the composition.

Specifically, the amount of the UV filter(s) in the composition according to the present invention may be from 5 to 60% by weight, preferably from 10 to 50% by weight, more preferably from 20 to 40% by weight, and even more preferably from 30 to 40% by weight, relative to the total weight of the composition.

### (Water)

The composition according to the present invention comprises (c) water. In the present invention, water forms the aqueous phase.

The amount of water may be 10% by weight or more, preferably 20% by weight or more, and more preferably 25% by weight or more, relative to the total weight of the composition.

The amount of the water may be 70% by weight or less, preferably 60% by weight or less, and more preferably 50% by weight or less, relative to the total weight of the composition according to the present invention.

Specifically, the amount of the water in the composition according to the present invention may be from 10 to 70% by weight, preferably from 20 to 60% by weight, and more preferably from 25 to 50% by weight, relative to the total weight of the composition.

### (Other Ingredients)

### • Oil

The composition according to the present invention may comprise at least one oil. If two or more oils are used, they may be the same or different.

Here, "oil" means a fatty compound or substance which is in the form of a liquid or a paste (non-solid) at room temperature (25°C) under atmospheric pressure (760 mmHg). As the oils, those generally used in cosmetics can be used alone or in combination thereof. These oils may be volatile or non-volatile. Preferably the oil is different from the UV filter.

The oil can form the oily phase of the composition according to the present invention.

The oil may be a non-polar oil such as a hydrocarbon oil, a silicone oil, or the like; a polar oil such as a plant or animal oil and an ester oil or an ether oil; or a mixture thereof.

The oil may be selected from the group consisting of oils of plant or animal origin, synthetic oils, silicone oils, hydrocarbon oils and fatty alcohols.

As examples of plant oils, mention may be made of, for example, linseed oil, camellia oil, macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, and mixtures thereof.

As examples of animal oils, mention may be made of, for example, squalene and squalane.

As examples of synthetic oils, mention may be made of alkane oils such as isododecane and isohexadecane, ester oils, ether oils, and artificial triglycerides.

The ester oils are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the present invention are derived is branched.

Among the monoesters of monoacids and of monoalcohols, mention may be made of ethyl palmitate, ethyl hexyl palmitate, isopropyl palmitate, dicaprylyl carbonate, alkyl myristates such as isopropyl myristate or ethyl myristate, isocetyl stearate, 2-ethylhexyl isononanoate, isononyl isononanoate, isodecyl neopentanoate, and isostearyl neopentanoate.

Esters of C₄-C₂₂ dicarboxylic or tricarboxylic acids and of C₁-C₂₂ alcohols, and esters of monocarboxylic, dicarboxylic, or tricarboxylic acids and of non-sugar C₄-C₂₆ dihydroxy, trihydroxy, tetrahydroxy, or pentahydroxy alcohols may also be used.

Mention may especially be made of: diethyl sebacate; isopropyl lauroyl sarcosinate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

As ester oils, one can use sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides, or polysaccharides.

Examples of suitable sugars that may be mentioned include sucrose (or saccharose), glucose, galactose, ribose, fucose, maltose, fructose, mannose, arabinose, xylose, and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may have one to three conjugated or non-conjugated carbon-carbon double bonds.

The esters according to this variant may also be selected from monoesters, diesters, triesters, tetraesters, and polyesters, and mixtures thereof.

These esters may be, for example, oleates, laurates, palmitates, myristates, behenates, cocoates, stearates, linoleates, linolenates, caprates, and arachidonates, or mixtures thereof such as, especially, oleopalmitate, oleostearate, and palmitostearate mixed esters, as well as pentaerythrityl tetraethyl hexanoate.

More particularly, use is made of monoesters and diesters and especially sucrose, glucose, or methylglucose monooleates or dioleates, stearates, behenates, oleopalmitates, linoleates, linolenates, and oleostearates.

An example that may be mentioned is the product sold under the name Glucate^{®} DO by the company Amerchol, which is a methylglucose dioleate.

As examples of preferable ester oils, mention may be made of, for example, diisopropyl adipate, dioctyl adipate, 2-ethylhexyl hexanoate, ethyl laurate, cetyl octanoate, octyldodecyl octanoate, isodecyl neopentanoate, myristyl propionate, 2-ethylhexyl 2-ethylhexanoate, 2-ethylhexyl octanoate, 2-ethylhexyl caprylate/caprate, methyl palmitate, ethyl palmitate, isopropyl palmitate, dicaprylyl carbonate, isopropyl lauroyl sarcosinate, isononyl isononanoate, ethylhexyl palmitate, isohexyl laurate, hexyl laurate, isocetyl stearate, isopropyl isostearate, isopropyl myristate, isodecyl oleate, glyceryl tri(2-ethylhexanoate), pentaerythrithyl tetra(2-ethylhexanoate), 2-ethylhexyl succinate, diethyl sebacate, and mixtures thereof.

As examples of ether oils, mention may be made of, for example, ether oils with a short hydrocarbon chain or chains, such as dicaprylyl ether.

As examples of artificial triglycerides, mention may be made of, for example, capryl caprylyl glycerides, glyceryl trimyristate, glyceryl tripalmitate, glyceryl trilinolenate, glyceryl trilaurate, glyceryl tricaprate, glyceryl tricaprylate, glyceryl tri(caprate/caprylate), and glyceryl tri(caprate/caprylate/linolenate).

As examples of silicone oils, mention may be made of, for example, linear organopolysiloxanes such as dimethylpolysiloxane, methylphenylpolysiloxane, methylhydrogenpolysiloxane, and the like; cyclic organopolysiloxanes such as cyclohexasiloxane, octamethylcyclotetrasiloxane, decamethylcyclopentasiloxane, dodecamethylcyclohexasiloxane, and the like; and mixtures thereof.

Preferably, the silicone oil is chosen from liquid polydialkylsiloxanes, especially liquid polydimethylsiloxanes (PDMS) and liquid polyorganosiloxanes comprising at least one aryl group.

These silicone oils may also be organomodified. The organomodified silicones that can be used in accordance with the present invention are silicone oils as defined above and comprise in their structure one or more organofunctional groups attached via a hydrocarbon-based group.

Organopolysiloxanes are defined in greater detail in Walter Noll's Chemistry and Technology of Silicones (1968), Academic Press. They may be volatile or non-volatile.

When they are volatile, the silicones are more particularly chosen from those having a boiling point of between 60°C and 260°C.

Non-volatile polydialkylsiloxanes may also be used. These non-volatile silicones are more particularly chosen from polydialkylsiloxanes, among which mention may be made mainly of polydimethylsiloxanes containing trimethylsilyl end groups.

Among these polydialkylsiloxanes, mention may be made, in a non-limiting manner, of the following commercial products:
- the Silbione^{®} oils of the 47 and 70 047 series or the Mirasil^{®} oils sold by Rhodia, for instance the oil 70 047 V 500 000;
- the oils of the Mirasil^{®} series sold by the company Rhodia;
- the oils of the 200 series from the company Dow Coming, such as DC200 with a viscosity of 60 000 mm²/s; and
- the Viscasil^{®} oils from General Electric and certain oils of the SF series (SF 96, SF 18) from General Electric.

Mention may also be made of polydimethylsiloxanes containing dimethylsilanol end groups known under the name dimethiconol (CTFA), such as the oils of the 48 series from the company Rhodia.

Among the silicones containing aryl groups, mention may be made of polydiarylsiloxanes, especially polydiphenylsiloxanes and polyalkylarylsiloxanes such as phenyl silicone oil.

Examples that may be mentioned include the products sold under the following names:
- the Silbione^{®} oils of the 70 641 series from Rhodia;
- the oils of the Rhodorsil^{®} 70 633 and 763 series from Rhodia;
- the oil Dow Coming 556 Cosmetic Grade Fluid from Dow Coming;
- the silicones of the PK series from Bayer, such as the product PK20;
- certain oils of the SF series from General Electric, such as SF 1023, SF 1154, SF 1250, and SF 1265.

As the phenyl silicone oil, phenyl trimethicone is preferable.

The organomodified liquid silicones may especially contain polyethyleneoxy and/or polypropyleneoxy groups. Mention may thus be made of the silicone KF-6017 proposed by Shin-Etsu, and the oils Silwet^{®} L722 and L77 from the company Union Carbide.

Hydrocarbon oils may be chosen from:
- linear or branched, optionally cyclic, C₆-C₁₆ lower alkanes. Examples that may be mentioned include hexane, undecane, dodecane, tridecane, and isoparaffins, for instance isohexadecane, isododecane, and isodecane; and
- linear or branched hydrocarbons containing more than 16 carbon atoms, such as liquid paraffins, liquid petroleum jelly, polydecenes and hydrogenated polyisobutenes such as Parleam^{®}, and squalane.

As preferable examples of hydrocarbon oils, mention may be made of, for example, linear or branched hydrocarbons such as isohexadecane, isododecane, squalane, mineral oil (e.g., liquid paraffin), paraffin, vaseline or petrolatum, naphthalenes, and the like; hydrogenated polyisobutene, isoeicosan, and decene/butene copolymer; and mixtures thereof.

The term "fatty" in the fatty alcohol means the inclusion of a relatively large number of carbon atoms. Thus, alcohols which have 4 or more, preferably 6 or more, and more preferably 12 or more carbon atoms are encompassed within the scope of fatty alcohols. The fatty alcohol may be saturated or unsaturated. The fatty alcohol may be linear or branched.

The fatty alcohol may have the structure R-OH wherein R is chosen from saturated and unsaturated, linear and branched radicals containing from 4 to 40 carbon atoms, preferably from 6 to 30 carbon atoms, and more preferably from 12 to 20 carbon atoms. In at least one embodiment, R may be chosen from C₁₂-C₂₀ alkyl and C₁₂-C₂₀ alkenyl groups. R may or may not be substituted with at least one hydroxyl group.

As examples of the fatty alcohol, mention may be made of lauryl alcohol, cetyl alcohol, stearyl alcohol, isostearyl alcohol, behenyl alcohol, undecylenyl alcohol, myristyl alcohol, octyldodecanol, hexyldecanol, oleyl alcohol, linoleyl alcohol, palmitoleyl alcohol, arachidonyl alcohol, erucyl alcohol, and mixtures thereof.

It is preferable that the fatty alcohol be a saturated fatty alcohol.

Thus, the fatty alcohol may be selected from straight or branched, saturated or unsaturated C₆-C₃₀ alcohols, preferably straight or branched, saturated C₆-C₃₀ alcohols, and more preferably straight or branched, saturated C₁₂-C₂₀ alcohols.

The term "saturated fatty alcohol" here means an alcohol having a long aliphatic saturated carbon chain. It is preferable that the saturated fatty alcohol be selected from any linear or branched, saturated C₆-C₃₀ fatty alcohols. Among the linear or branched, saturated C₆-C₃₀ fatty alcohols, linear or branched, saturated C₁₂-C₂₀ fatty alcohols may preferably be used. Any linear or branched, saturated C₁₆-C₂₀ fatty alcohols may be more preferably used. Branched C₁₆-C₂₀ fatty alcohols may be even more preferably used.

As examples of saturated fatty alcohols, mention may be made of lauryl alcohol, cetyl alcohol, stearyl alcohol, isostearyl alcohol, behenyl alcohol, undecylenyl alcohol, myristyl alcohol, octyldodecanol, hexyldecanol, and mixtures thereof. In one embodiment, cetyl alcohol, stearyl alcohol, octyldodecanol, hexyldecanol, or a mixture thereof (e.g., cetearyl alcohol) as well as behenyl alcohol, can be used as a saturated fatty alcohol.

According to at least one embodiment, the fatty alcohol used in the composition according to the present invention is preferably chosen from cetyl alcohol, octyldodecanol, hexyldecanol, and mixtures thereof.

It is preferable that the oil be chosen from hydrocarbon oils, ester oils, silicone oils, and mixtures thereof.

In one embodiment, it is preferable that the oil be selected from the group consisting of mineral oil, octyldodecanol, petrolatum, isododecane, hydrogenated polyisobutene, isopropyl myristate, isononyl isononanoate, dimethicone, cyclohexasiloxane, C₂₀₋C₂₂ alcohol, cetyl palmitate, oleyl alcohol, cetyl alcohol, and mixtures thereof.

The amount of the oil in the composition according to the present invention may range from 1 to 50% by weight, preferably from 5 to 40% by weight, and more preferably from 5 to 30% by weight, relative to the total weight of the composition.

### • Cosmetically Acceptable Hydrophilic Organic Solvent

The composition according to the present invention may comprise at least one cosmetically acceptable hydrophilic organic solvent. If two or more oils are used, they may be the same or different.

The cosmetically acceptable hydrophilic organic solvent can form the aqueous phase of the composition according to the present invention.

The cosmetically acceptable hydrophilic organic solvent(s) may include, for example, substantially linear or branched lower mono-alcohols having from 1 to 8 carbon atoms, such as ethanol, propanol, butanol, isopropanol, and isobutanol; aromatic alcohols, such as benzyl alcohol and phenylethyl alcohol; polyols or polyol ethers, such as propylene glycol, dipropylene glycol, isoprene glycol, butylene glycol, glycerine, propanediol, caprylyl glycol, sorbitol, ethylene glycol monomethyl, monoethyl and monobutyl ethers, propylene glycol ethers, such as propylene glycol monomethylether, diethylene glycol alkyl ethers, such as diethylene glycol monoethylether or monobutylether; polyethylene glycols, such as PEG-4, PEG-6, and PEG-8, and their derivatives.

The amount of the cosmetically acceptable hydrophilic organic solvent(s) in the composition according to the present invention may range from 0.1% to 20% by weight, preferably from 1% to 15% by weight, and more preferably from 2% to 10% by weight, relative to the total weight of the composition.

### • Adjuvants

The compositions according to the present invention may also contain various adjuvants conventionally used in compositions for sun care products, which may be selected from a physiologically acceptable medium, cationic, anionic, non-ionic, amphoteric or zwitterionic polymers, or mixtures thereof, cationic, anionic, non-ionic, amphoteric or zwitterionic surfactants or emulsifiers, or mixtures thereof, antioxidants, such as tocopherol, neutralizing agents, such as triethanolamine, sequestering agents, such as disodium EDTA, fragrances, dispersing agents, dyes and/or pigments, such as VIOLET 2 and GREEN 6, film-forming agents and/or thickeners other than the (a) semi-crystalline or crystalline oil thickener, ceramides, preservatives, such as phenoxy ethanol, co-preservatives and opacifying agents.

In one preferred embodiment of the present invention, the composition according to the present invention comprises cationic, anionic, non-ionic, amphoteric or zwitterionic surfactants or emulsifiers in an amount of 3% by weight or less, preferably 2% by weight or less, and more preferably 1 % by weight or less, relative to the total amount of the composition.

In one particular embodiment of the present invention, the composition according to the present invention is free of any kind of surfactant or emulsifier.

The adjuvants may be present in the composition of the present invention in an amount preferably ranging from 0.01% to 30% by weight, preferably from 0.1% to 20% by weight, and more preferably from 1% to 10% by weight, relative to the total weight of the composition.

### [Preparation]

The composition according to the present invention can be prepared by mixing the ingredients (a) to (c), as essential ingredients, as well as optional ingredient(s), as explained above.

The method and means to mix the above essential and optional ingredients are not limited. Any conventional method and means can be used to mix the above essential and optional ingredients to prepare the composition according to the present invention. In general, the aqueous phase and the oily phase are prepared separately, and then they are mixed to prepare the composition according to the present invention. The mix ratio of the aqueous phase and the oily phase are not particularly limited, but generally from 1:5 to 5:1, preferably from 1:3 to 3:1, and more preferably from 1:2 to 2:1. It is also possible to heat the ingredients, for example, to 50 to 90°C, to ensure that they are dissolved completely during the method.

### [Cosmetic Process]

The composition according to the present invention may preferably be used as a cosmetic composition. The cosmetic composition may be a sun care composition for protecting keratinous substances, such as skin, from UV rays.

Therefore, the present invention relates to a composition as defined in the claims for use in a process for protecting keratinous substances, preferably skin, from UV radiation, which is applied to the keratinous substances. The process according to the present invention can further comprise another step of mixing the aqueous phase and the oily phase of the present invention prior to the application on the keratinous substance, so as to be a homogenous composition.

### [Product]

The present invention also relates to a cosmetic product comprising the composition according to the present invention. The product according to the present invention comprises the composition according to the present invention and a container or a package in which the composition is contained.

The container or package is not particularly limited; however, since the composition according to the present invention has a relatively low viscosity, there is a benefit in that the composition can be applied without using an aerosol device. Therefore, the container or package are preferably selected from non-aerosol devices, such as a bottle, a tube, a vessel, or a container equipped with a mist generating means, such as a mist-pump, a spray bottle, and a pump bottle.

### EXAMPLES

The present invention will be described in a more detailed manner by way of examples.

### [Compositions]

Each of the compositions according to Examples 1 to 3 (Ex. 1 to Ex. 3) and Comparative Examples 1 to 5 (Comp. Ex. 1 to Comp. Ex. 5) was prepared in accordance with the following preparation protocol. The compositions are shown in the following Table 1.

Among the ingredients, polystearyl acrylate was obtained from the company Air Products (product name: Intelimer^{®} IPA 13-1) and the polymethylene wax was obtained from Cirebelle (product name: Cirebelle 303). The numerical values for the amounts of the ingredients are all based on "% by weight" as active raw materials.

### [Preparation Protocol]

1) Mixing water, disodium EDTA, glycerin, caprylyl glycol, propanediol, phenylbenzimidazole sulfonic acid, terephthalylidene dicamphor sulfonic acid, and Violet 2 and Green 6, if present, at 75°C, and then cooling them down to room temperature, adding ethanol and triethanol amine, and mixing them to prepare an aqueous phase.
2) Mixing butyl methoxydibenzoylmethane, ethylhexyl salicylate, ethylhexyl triazone, octocrylene, homosalate, bis-ethylhexyloxyphenol, methoxyphenyl triazine, tocopherol dicaprylyl ether, diisopropyl sebacate, and dicaprylyl carbonate, and polystearyl acrylate or polymethylene wax, if present, at 75°C, and then cooling them down to room temperature, adding fragrance, and mixing them to prepare an oily phase. The viscosity of each of the oily phases was measured with the following evaluation protocol for viscosity.
3) Mixing the obtained aqueous phase and oily phase at a 1:1 weight ratio to obtain each of the compositions according to Examples 1 to 3 and Comparative Examples 1 to 5.

### [Evaluation]

### (Viscosity)

Each of the oily phases of Examples 1 to 3 and Comparative Examples 1 to 5 was measured with VISCOMAN^{™}(GILSON Technology) at room temperature. The bulk viscosity (mPaS, PaS) was automatically calculated from the needed aspiration force in the small pipet (10 µL). The viscosity was measured 3 times and its average data was used.

### (Film Homogeneity)

Each of the compositions was shaken 10 times in a capped container to be homogeneous. Each of the homogenous compositions was applied to a polypropylene sheet (thickness: 0.2 mm, PX-P from Sekisui Kagaku) using an applicator called "Elecometer 4340" with a force of 1kgf, so as to obtain 10 µm of coating of the composition on the sheet. The sheet was then exposed to a UV lamp (UV black ray B-100AP, wave length: 365nm) and a picture was taken. The film homogeneity was evaluated based on the visual appearance of the coated sheet in five grades (Very Good, Good, Fair, Bad, and Very Bad). If the appearance was homogeneous intensive black, it was ranked as "Very Good". If the appearance was non-homogenous blue translucent, it was ranked as "Very Bad).

### (SPF)

Each of the compositions was shaken 10 times in a capped container to be homogeneous. Each of the homogenous compositions was transferred onto a plate (Helio plate HD 6, PMMA, roughness: 6µm) with an adjustable pipette in an amount of 0.8 mg/cm² of the plate and then uniformly spread by finger. The coated plate was air dried for 15 minutes at room temperature. The obtained sample plate was placed into a Labsphere Ultraviolet Transmittance Analyzer (Model UV-2000 from Solar Light Company, Philadelphia, Pennsylvania). Irradiation took place at 12 points on the sample plate. The in vitro SPF level was estimated from absorbance intensity at 310 nm.

### (Mist Quality)

Each of the compositions was shaken 10 times in a spray bottle to be homogeneous. The mist quality of each of the homogeneous compositions was evaluated visually based on its sprayed droplet size and spray angle. If the sprayed droplet size was fine and the spray angle was wide, it was scored as "Good". If the sprayed droplet size was big and the spray angle was narrow, it was scored as "Bad". In this evaluation, ALBEA mechanical pump was used as a standard spray bottle.

The evaluation results are summarized in Table 2 below.

**Table 1**

| | Ingredients | Ex.1 | Ex.2 | Ex.3 | Comp. Ex.1 | Comp. Ex.2 | Comp. Ex.3 | Comp. Ex.4 | Comp. Ex.5 |
|---|---|---|---|---|---|---|---|---|---|
| Aqueous Phase | Water | qs 100 | qs 100 | qs 100 | qs 100 | qs 100 | qs 100 | qs 100 | qs 100 |
| | Disodium EDTA | 0.1 | 0.1 | 0.1 | 0.1 | 0.1 | 0.1 | 0.1 | 0.1 |
| | Violet 2 | - | 0.00009 | - | - | - | - | - | - |
| | Green 6 | - | 0.00014 | - | - | - | - | - | - |
| | Phenoxyethanol | 0.5 | 0.5 | 0.5 | 0.5 | 0.5 | 0.5 | 0.5 | 0.5 |
| | Glycerin | 3 | 3 | 3 | 3 | 3 | 3 | 3 | 3 |
| | Caprylyl Glycol | 0.3 | 0.3 | 0.3 | 0.3 | 0.3 | 0.3 | 0.3 | 0.3 |
| | Propanediol | 3 | 3 | 3 | 3 | 3 | 3 | 3 | 3 |
| | Phenylbenzimidazole Sulfonic Acid | 3 | 1 | 3 | 3 | 3 | 3 | 3 | 3 |
| | Terephthalylidene Dicamphor Sulfonic Acid | 0.9 | 0.9 | 0.9 | 0.9 | 0.9 | 0.9 | 0.9 | 0.9 |
| | Ethanol | 4 | 3 | 4 | 4 | 4 | 4 | 4 | 4 |
| | Triethanolamine | 1.9 | 0.8 | 1.9 | 1.9 | 1.9 | 1.9 | 1.9 | 1.9 |
| Oily Phase | Butyl Methoxydibenzoylmethane | 4 | 4 | 4 | 4 | 4 | 4 | 4 | 4 |
| | Ethylhexyl Salicylate | 4.5 | 4.5 | 4.5 | 4.5 | 4.5 | 4.5 | 4.5 | 4.5 |
| | Ethylhexyl Triazone | 5 | 3 | 5 | 5 | 5 | 5 | 5 | 5 |
| | Octocrylene | 5.5 | 5.5 | 5.5 | 5.5 | 5.5 | 5.5 | 5.5 | 5.5 |
| | Homosalate | 9 | 9 | 9 | 9 | 9 | 9 | 9 | 9 |
| | Bis-ethylhexyloxyphenol Methoxyphenyl Triazine | 1.5 | 1.5 | 1.5 | 1.5 | 1.5 | 1.5 | 1.5 | 1.5 |
| | Tocopherol | 0.1 | 0.1 | 0.1 | 0.1 | 0.1 | 0.1 | 0.1 | 0.1 |
| | Dicaprylyl Ether | 9 | 9 | 9 | 9 | 9 | 9 | 9 | 9 |
| | Diisopropyl Sebacate | 6 | 2 | 6 | 6 | 6 | 6 | 6 | 6 |
| | Dicaprylyl Carbonate | 5.3 | 11 | 5.3 | 5.3 | 5.3 | 5.3 | 5.3 | 5.3 |
| | Polystearyl Acrylate | 0.1 | 0.1 | - | - | - | - | - | - |
| | Polymethylene Wax | - | - | 0.1 | - | - | - | - | - |
| | Dextrin Palmitate | - | - | - | - | 0.1 | - | - | - |
| | Dibutyl Lauroyl Glutamide | - | - | - | - | - | 0.1 | - | - |
| | Ethylcellulose | - | - | - | - | - | - | 0.1 | - |
| | Ethylenediamine/Stearyl Dimer Dilinoleate Copolymer | - | - | - | - | - | - | - | 0.1 |
| | Fragrance | - | 0.3 | - | - | - | - | - | - |

| Evaluation | | Ex.1 | Ex.2 | Ex.3 | Comp. Ex.1 | Comp. Ex.2 | Comp. Ex.3 | Comp. Ex.4 | Comp. Ex.5 |
|---|---|---|---|---|---|---|---|---|---|
| Film Homogeneity | | Good | Good | Good | Poor | Poor | Poor | Poor | Poor |
| Absorbance intensity at 310nm | | 1.4 | 1.2 | 1.4 | 0.8 | 0.8 | 0.8 | 0.8 | 0.8 |
| Mist Quality | | Good | Good | Good | Good | Good | Good | Good | Good |
| Viscosity of the Oily Phase at Room Temperature (mPa·s) | | 35 | 30 | 30 | 20 | 35 | 25 | 35 | 30 |

As can be seen from Table 2, the compositions according to each of Examples 1 to 3, which include the specific combination of the ingredients (a) to (c) of the present invention exhibited a good film homogeneity and thus a high absorbance intensity at 310nm.

On the other hand, each of the compositions according to Comparative Examples 1 to 5, which lacks semi-crystalline or crystalline lipophilic thickeners as defined in claim 1, showed inferior film homogeneity and thus a low absorbance intensity at 310nm.

Therefore, it can be concluded that the composition according to the present invention can be very preferable as a sun care composition, since it has a strong UV protecting effect due to high in vitro SPF level. In addition, since the mixed homogenous composition of the present invention has a relatively low viscosity, it can be easily applied onto keratinous substances. Furthermore, the transparent appearance of the present composition meets the preference of the consumers.

## Claims

1. A bi-phase composition comprising an aqueous phase and an oily phase, wherein the composition comprises:
(a) at least one semi-crystalline or crystalline lipophilic thickener selected from poly C₁₀₋C₃₀ alkyl acrylate and crystalline apolar hydrocarbon-based waxes constituted solely of carbon and hydrogen atoms,
(b) at least one UV filter, and
(c) water, and
wherein the aqueous phase and the oily phase are visually separated at rest.

2. The composition according to Claim 1, wherein the viscosity of the oily phase is more than 5 mPa-s, the viscosity being measured by by the method according to the description.

3. The composition according to Claim 1, wherein the crystalline wax is selected from polymethylene wax.

4. The composition according to any one of the preceding claims, wherein the (b) UV filter comprises at least one lipophilic organic UV filter.

5. The composition according to Claim 4, wherein the (b) UV filter further comprises at least one hydrophilic organic UV filter.

6. The composition according to any one of the preceding claims, wherein the amount of the semi-crystalline or crystalline lipophilic thickener(s) in the composition is from 0.001 to 15% by weight, preferably from 0.01 to 5% by weight, and more preferably from 0.05 to 2% by weight, relative to the total weight of the composition.

7. The composition according to any one of the preceding claims, comprising at least one oil.

8. The composition according to any one of the preceding claims, comprising cationic, anionic, non-ionic, amphoteric or zwitterionic surfactants or emulsifiers in an amount of 3% by weight or less, preferably 2% by weight or less, and more preferably 1% by weight or less, relative to the total amount of the composition.

9. The composition according to any one of the preceding claims, which is free of any kind of surfactant or emulsifier.

10. The composition according to any one of preceding claims, which is transparent.

11. The composition according to any one of the preceding claims, for use in a process for protecting keratinous substances, preferably skin, from UV radiation, which is applied to the keratinous substances.

12. A product comprising the composition according to any one of Claims 1 to 10 and a container selected from a bottle, a tube, a vessel, or a container equipped with a mist-pump, a spray bottle, and a pump bottle.

## Patentansprüche

1. Zweiphasige Zusammensetzung, die eine wässrige Phase und eine ölige Phase umfasst, wobei die Zusammensetzung Folgendes umfasst:
(a) wenigstens ein teilkristallines oder kristallines lipophiles Verdickungsmittel, das aus Poly-C₁₀-C₃₀-Alkyl-Acrylat und kristallinen apolaren Wachsen auf Kohlenwasserstoffbasis, die nur aus Kohlenstoff- und Wasserstoffatomen gebildet sind, ausgewählt ist,
(b) wenigstens einen UV-Filter und
(c) Wasser, und
wobei die wässrige Phase und die ölige Phase in Ruhe visuell getrennt vorliegen.

2. Zusammensetzung nach Anspruch 1, wobei die Viskosität der öligen Phase mehr als 5 mPa·s beträgt, wobei die Viskosität mit dem Verfahren gemäß der Beschreibung gemessen wird.

3. Zusammensetzung nach Anspruch 1, wobei das kristalline Wachs aus Polymethylen-Wachs ausgewählt ist.

4. Zusammensetzung nach einem der vorhergehenden Ansprüche, wobei der (b) UV-Filter wenigstens einen lipophilen organischen UV-Filter umfasst.

5. Zusammensetzung nach Anspruch 4, wobei der (b) UV-Filter ferner wenigstens einen hydrophilen organischen UV-Filter umfasst.

6. Zusammensetzung nach einem der vorhergehenden Ansprüche, wobei die Menge des/r teilkristallinen oder kristallinen lipophilen Verdickungsmittel(s) in der Zusammensetzung 0,001 bis 15 Gew.-%, vorzugsweise 0,01 bis 5 Gew.-% und besonders bevorzugt 0,05 bis 2 Gew.-%, bezogen auf das Gesamtgewicht der Zusammensetzung, beträgt.

7. Zusammensetzung nach einem der vorhergehenden Ansprüche, die wenigstens ein Öl umfasst.

8. Zusammensetzung nach einem der vorhergehenden Ansprüche, die kationische, anionische, nichtionische, amphotere oder zwitterionische Tenside oder Emulgatoren in einer Menge von 3 Gew.-% oder weniger, vorzugsweise 2 Gew.-% oder weniger und besonders bevorzugt 1 Gew.-% oder weniger, bezogen auf die Gesamtmenge der Zusammensetzung, umfasst.

9. Zusammensetzung nach einem der vorhergehenden Ansprüche, die frei von jeder Art von Tensid oder Emulgator ist.

10. Zusammensetzung nach einem der vorhergehenden Ansprüche, die transparent ist.

11. Zusammensetzung nach einem der vorhergehenden Ansprüche zur Verwendung in einem Verfahren zum Schützen von keratinhaltigen Substanzen, vorzugsweise Haut, vor UV-Strahlung, die auf die keratinhaltigen Substanzen aufgetragen wird.

12. Produkt, das die Zusammensetzung nach einem der Ansprüche 1 bis 10 und einen Behälter, ausgewählt aus einer Flasche, einer Tube, einem Gefäß oder einem Behälter, der mit einer Sprühnebelpumpe versehen ist, einer Sprühflasche und einer Pumpflasche, umfasst.

## Revendications

1. Composition biphase comprenant une phase aqueuse et une phase huileuse, dans laquelle la composition comprend :
(a) au moins un épaississant lipophile semi-cristallin ou cristallin sélectionné parmi le poly(acrylate d'alkyle en C₁₀-C₃₀) et des cires à base d'hydrocarbures apolaires cristallines consistant exclusivement en des atomes de carbone et d'hydrogène,
(b) au moins un filtre UV, et
(c) de l'eau, et
dans laquelle la phase aqueuse et la phase huileuse sont séparées visuellement au repos.

2. Composition selon la revendication 1, dans laquelle la viscosité de la phase huileuse est supérieure à 5 mPa·s, la viscosité étant mesurée par le procédé conformément à la description.

3. Composition selon la revendication 1, dans laquelle la cire cristalline est sélectionnée parmi une cire de polyméthylène.

4. Composition selon l'une quelconque des revendications précédentes, dans laquelle le (b) filtre UV comprend au moins un filtre UV organique lipophile.

5. Composition selon la revendication 4, dans laquelle le (b) filtre UV comprend en outre au moins un filtre UV organique hydrophile.

6. Composition selon l'une quelconque des revendications précédentes, dans laquelle la quantité du ou des épaississants lipophiles semi-cristallins ou cristallins dans la composition va de 0,001 à 15 % en poids, de préférence de 0,01 à 5 % en poids, et plus préférentiellement de 0,05 à 2 % en poids par rapport au poids total de la composition.

7. Composition selon l'une quelconque des revendications précédentes, comprenant au moins une huile.

8. Composition selon l'une quelconque des revendications précédentes, comprenant des tensioactifs ou des émulsifiants cationiques, anioniques, non-ioniques, amphotères ou zwitterioniques en une quantité inférieure ou égale à 3 % en poids, de préférence inférieure ou égale à 2 % en poids, et plus préférentiellement inférieure ou égale à 1 % en poids, par rapport à la quantité totale de la composition.

9. Composition selon l'une quelconque des revendications précédentes, qui est exempte de tout type de tensioactif ou d'émulsifiant.

10. Composition selon l'une quelconque des revendications précédentes, qui est transparente.

11. Composition selon l'une quelconque des revendications précédentes, pour utilisation dans un processus pour protéger les substances kératineuses, de préférence la peau, du rayonnement UV, qui est appliquée sur les substances kératineuses.

12. Produit comprenant la composition selon l'une quelconque des revendications 1 à 10 et contenant sélectionné parmi un flacon, un tube, un récipient, ou un contenant équipé d'une pompe de brumisation, un flacon pulvérisateur, et un flacon-pompe.
